(19) Europäisches Patentamt
European Patent Office
Office européen des brevets

(11) Veröffentlichungsnummer: 0 415 889 A2

(12) # EUROPÄISCHE PATENTANMELDUNG

(21) Anmeldenummer: 90810641.2

(22) Anmeldetag: 23.08.90

(51) Int. Cl.5: C07D 239/54, C07D 239/22, A01N 43/54, A61K 31/505

(30) Priorität: 01.09.89 CH 3173/89

(43) Veröffentlichungstag der Anmeldung: 06.03.91 Patentblatt 91/10

(84) Benannte Vertragsstaaten: AT BE CH DE DK ES FR GB GR IT LI LU NL SE

(71) Anmelder: CIBA-GEIGY AG
Klybeckstrasse 141
CH-4002 Basel(CH)

(72) Erfinder: Molleyres, Louis-Pierre, Dr.
Walter-Fürst-Strasse 5
CH-4102 Binningen(CH)

(54) **Anthelmintika.**

(57) Es werden neue Anthelmintika beschrieben, die als Wirkstoff eine Verbindung der Formel I

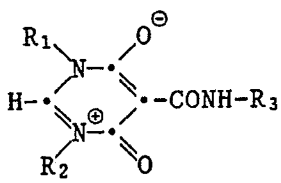

(I)

oder in ihrer hydrierten Form der Formel Ia

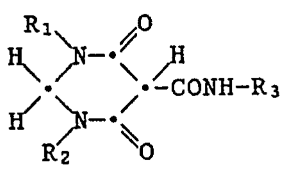

(Ia)

worin
$R_1$ und $R_2$ unabhängig voneinander für $C_1$-$C_6$-Alkyl, Allyl, $C_3$-$C_6$-Cycloalkyl oder Phenyl stehen; und $R_3$ für unsubstituiertes oder ein- oder mehrfach substituiertes Phenyl, Biphenylyl oder Phenoxyphenyl steht; wobei deren Substituenten ausgewählt sind aus der Gruppe Halogen, Cyano, $C_1$-$C_3$-Alkyl, $C_1$-$C_3$-Haloalkyl, $C_1$-$C_3$-Alkoxy, $C_1$-$C_3$-Haloalkoxy, $C_1$-$C_3$-Cyanoalkyl, Nitro, Amino oder durch $C_1$-$C_3$-Alkoxycarbonyl substituiertes $C_1$-$C_3$-Alkyl, unter Einschluss deren tautomeren Formen und physiologisch verträglichen Salze, enthalten. Es wird auch die Herstellung, Formulierung und Verwendung dieser Wirkstoffe beschrieben.

EP 0 415 889 A2

# ANTHELMINTIKA

Die vorliegende Erfindung betrifft neue 1,3-disubstituierte 5-Arylcarbamoyl-4(6)-oxo-6(4)-oxido-pyrimidinium-Betaine der nachstehenden Formel I und deren hydrierte Analoga der nachstehenden Formel Ia mit anthelmintischer Wirksamkeit; besagte Substanzen zur Verwendung in einem Verfahren zur Bekämpfung von Helminthen; Anthelmintische Mittel, die diese Substanzen als Wirkstoffe enthalten; die Herstellung der Wirkstoffe und Mittel; sowie die Verwendung der Wirkstoffe bzw. Mittel zur Bekämpfung von Helminthen, insbesondere von Nematoden, Cestoden und Trematoden, bei Warmblütern, vor allem Haus- und Nutztieren, insbesondere solchen aus der Klasse der Säugetiere.

Die erfindungsgemässen Verbindungen haben die Formel I

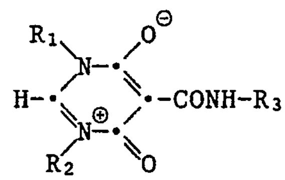

(I)

und in ihrer hydrierten Form die Formel (Ia)

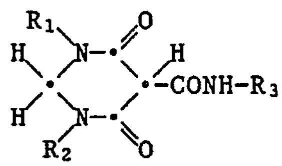

(Ia)

worin

$R_1$ und $R_2$ unabhängig voneinander für $C_1$-$C_6$-Alkyl, Allyl, $C_3$-$C_6$-Cycloalkyl oder Phenyl stehen; und

$R_3$ für unsubstituiertes oder ein- oder mehrfach substituiertes Phenyl, Biphenylyl oder Phenoxyphenyl steht; wobei deren Substituenten ausgewählt sind aus der Gruppe Halogen, Cyano, $C_1$-$C_3$-Alkyl, $C_1$-$C_3$-Haloalkyl, $C_1$-$C_3$-Alkoxy, $C_1$-$C_3$-Haloalkoxy, $C_1$-$C_3$-Cyanoalkyl, Nitro, Amino oder durch $C_1$-$C_3$-Alkoxycarbonyl substituiertes $C_1$-$C_3$-Alkyl, unter Einschluss ihrer tautomeren Formen und physiologisch verträglichen Salze.

In Formel I ist nur eine der zahlreichen möglichen Betainstrukturen wiedergegeben. Es kommen jedoch weitere isoelektronische Strukturformeln in Betracht, wie beispielsweise:

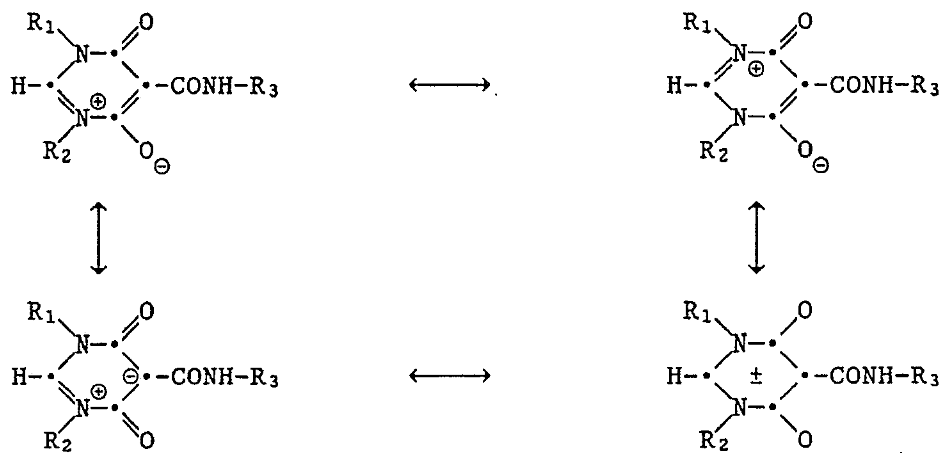

oder summarisch:

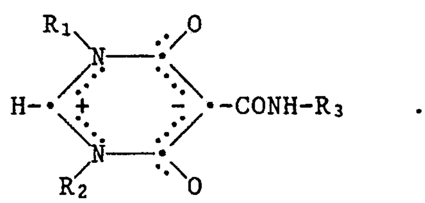

Die Verbindungen der Formel Ia können beispielsweise in folgenden tautomeren Formen vorliegen:

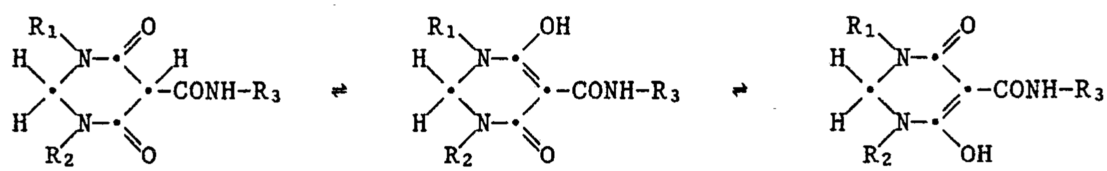

Die Verbindungen der Formeln I und Ia sind in allen Formen Bestandteil der vorliegenden Erfindung.

Unter dem Begriff Alkyl als Substitutent oder als Bestandteil eines Substituenten sind im Rahmen vorliegender Erfindung je nach Anzahl der angegebenen Kohlenstoffatome beispielsweise folgende geradkettige und verzweigte Gruppen zu verstehen: Methyl, Ethyl, n-Propyl, iso-Propyl, n-Butyl, sek.-Butyl, tert.Butyl und Isobutyl. Haloalkyl als Substituent oder als Bestandteil von Haloalkoxy steht für einen einfach bis perhalogenierten Alkylsubstituenten, wie beispielsweise für $CH_2Cl$, $CHCl_2$, $CCl_3$, $CH_2F$, $CHF_2$, $CF_3$, $CH_2Br$, $CHBr_2$, $CB_3$, $CH_2J$, $CJ_3$, $CHClF$, $CHBrCl$, $CFBrCl$, $C_2F_5$, $CH_2CH_2Cl$, $CHClCH_3$, $C_2Cl_5$ und $CHFCHCl_2$, vorzugsweise für $CF_3$. Unter Halogen soll hier und im folgenden Fluor, Chlor, Brom oder Jod, vorzugsweise Fluor, Chlor oder Brom, vor allem jedoch Chlor verstanden werden.

Cycloalkyl als Substituent oder als Bestandteil eines Substituenten steht je nach Anzahl der angegebenen Kohlenstoffatome beispielsweise für Cyclopropyl, Cyclobutyl, Cyclopentyl und Cyclohexyl. Cyanoalkyl steht für eine Alkylgruppe, worin ein Wasserstoffatom durch CN substituiert ist, vorzugsweise für eine Alkylgruppe, worin sich die CN-Gruppe am terminalen Kohlenstoffatom befindet.

Bei einer substituierten Biphenylylgruppe ergeben sich bezüglich der Ringzuordnung der Substituenten folgende Möglichkeiten:

a) Substituenten an demjenigen Phenylring, welcher direkt mit der Carbamoylgruppe verbunden ist (Substituenten ohne Apostroph),

b) Substitution an demjenigen Phenylring, welcher mit dem vorstehend unter a) angeführten Phenylring verbunden ist (Substituenten mit Apostroph) und

c) Substituenten an beiden Phenylringen.

Wird im Rahmen der vorliegenden Erfindung von einer Substitution "am Phenyl" gesprochen, so schliesst dieser Ausdruck alle unter a), b) und c) angegebenen Möglichkeiten ein. Als bevorzugt ist eine Substitution am unter a) angegebenen Phenylring anzusehen.

Bei den substituierten Phenoxyphenylgruppen kann a) der mit der Carbamoylgruppe verbundene

Phenylring substituiert sein oder b) der über Sauerstoff mit dem unter a) angeführten Phenylring verknüpfte Phenylring (Phenoxygruppe) oder es können beide Ringe substituiert sein. Auch hier schliesst die Substitution "am Phenyl" alle Möglichkeiten ein. Bevorzugt ist die Substitution gemäss b) am Ring der Phenoxygruppe.

Unter den Begriff physiologisch verträgliche Salze von Verbindungen der Formeln I und Ia fallen die Alkalimetall-, Ammonium- oder Aminsalze, wobei Natrium-, Kalium-, Ammonium- oder Alkylaminsalze, insbesondere Triethylaminsalze, bevorzugt sind. Es sind darunter aber auch die Additionssalze anorganischer und organischer Säuren zu verstehen, die durch Anlagerung einer equivalenten Menge einer salzbildenden Säure an das Basismolekül entstehen.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie z.B. Essigsäure, Trifluoressigsäure, Trichloressigsäure, Propionsäure, Glykolsäure, Thiocyansäure, Milchsäure, Bernsteinsäure, Zitronensäure, Ameisensäure, Benzolsulfonsäure, p-Toluolsulfonsäure, Methansulfonsäure, Salicylsäure, p-Aminosalicylsäure, Phthalsäure, 2-Phenoxybenzoesäure oder 2-Acetoxybenzoesäure.

Die Verbindungen der Formeln I und Ia liegen bei Raumtemperatur überwiegend als stabile Feststoffe vor, die einen Schmelzpunkt von ca. 100° bis ca. 300° C aufweisen. Sie besitzen sehr wertvolle anthelmintische Eigenschaften und lassen sich zur kurativen und präventiven Bekämpfung einer Reihe von Wurmerkrankungen bei Warmblütern, insbesondere bei Haus- und Nutztieren, vor allem aus der Klasse der Säugetiere einsetzen.

Nachfolgende Gruppen von Verbindungen der Formeln I und Ia sind bevorzugt:

(Iα) Verbindungen der Formeln I und Ia worin $R_1$ und $R_2$ unabhängig voneinander für Methyl, Ethyl, Isopropyl, Allyl oder Cyclopropyl stehen;
und $R_3$ für ein- bis dreifach substituiertes Phenyl steht, wobei die Substituenten am Phenyl ausgewählt sind aus der Gruppe Fluor, Chlor, Brom, Methyl, Ethyl, Nitro, Amino, Trifluormethyl, Methoxy, Ethoxy, $CH_2CN$ oder $-CH(CH_3)COOCH_3$.

(Iβ) Verbindungen der Formeln I und Ia worin $R_1$ und $R_2$ unabhängig voneinander für Methyl, Ethyl, Isopropyl, Allyl oder Cyclopropyl stehen;
und $R_3$ für ein- bis dreifach substituiertes Phenoxyphenyl steht, wobei die Substituenten am Phenyl ausgewählt sind aus der Gruppe Fluor, Chlor, Brom, Methyl, Ethyl, Nitro, Amino, Trifluormethyl, Methoxy, Ethoxy, $CH_2CN$ oder $-CH(CH_3)COOCH_3$.

Im Rahmen der Untergruppe (Iβ) sind die para-Phenoxyphenylvertreter besonders bevorzugt, und unter diesen diejenigen, bei denen die Phenoxyphenylgruppe ein- bis dreifach, vorzugsweise einfach, durch einen Substituenten aus der Gruppe Fluor, Chlor, $CF_3$, $OCF_3$ oder $CH_2CN$ substituiert ist.

(Iγ) Verbindungen der Formeln I und Ia worin $R_1$ und $R_2$ unabhängig voneinander für Methyl, Ethyl, Isopropyl, Allyl oder Cyclopropyl stehen;
und $R_3$ für ein- bis dreifach substituiertes Biphenylyl steht, wobei die Substituenten am Phenyl ausgewählt sind aus der Gruppe Fluor, Chlor, Brom, Methyl, Ethyl, Nitro, Amino, Trifluormethyl, Methoxy, Ethoxy, $CH_2CN$ und $-CH(CH_3)COOCH_3$.

Besonders bevorzugt im Rahmen der Untergruppe (Iγ) sind jene Vertreter, bei denen die Biphenylylgruppe in 4-Position an die Carbamoylgruppe gebunden ist und der Biphenylylrest unsubstituiert oder ein- bis dreifach, vorzugsweise einfach, durch einen Substituenten ausgewählt aus der Gruppe Fluor, Chlor, $CF_3$, $OCF_3$ und $CH_2CN$ substituiert ist.

Ferner sind folgende Gruppen bevorzugt:

a) Verbindungen der Formeln I und Ia, worin $R_1$ und $R_2$ unabhängig voneinander für Methyl, Ethyl oder Allyl stehen und $R_3$ für unsubstituiertes oder durch einen oder zwei Substituenten, ausgewählt aus der Gruppe Fluor, Chlor, Methyl und Methoxy, substituiertes Phenyl, für unsubstituiertes oder durch Methoxy monosubstituiertes Biphenylyl oder für durch Trifluormethyl monosubstituiertes Phenoxyphenyl steht;

b) Verbindungen der Formeln I und Ia, worin einer der Substituenten $R_1$ und $R_2$ für Methyl und der andere für Methyl, Ethyl oder Allyl steht und $R_3$ für unsubstituiertes oder durch Fluor oder Chlor mono- oder disubstituiertes oder durch Methyl oder Methoxy monosubstituiertes Phenyl, für unsubstituiertes oder durch Methoxy monosubstituiertes 3- oder 4-Biphenylyl oder für (Trifluormethylphenoxy)-phenyl steht;

c) Verbindungen der Formeln I und Ia, worin $R_1$ und $R_2$ jeweils für Methyl stehen und $R_3$ für unsubstituiertes oder durch Fluor oder Chlor mono- oder disubstituiertes Phenyl oder für (Trifluormethylphenoxy)-phenyl steht;

d) Verbindungen der Formel I, worin $R_1$ und $R_2$ jeweils für Methyl stehen und $R_3$ für unsubstituiertes oder durch Fluor oder Chlor mono- oder disubstituiertes Phenyl steht.

Besonders bevorzugte Einzelvertreter der Formel I sind:
1,3-Dimethyl-5-[3,4-dichlorphenylcarbamoyl]-4(6)-oxo-6(4)-oxido-(1H,5H)-pyrimidiniumbetain;
1,3-Dimethyl-5-[2,4-difluorphenylcarbamoyl]-4(6)-oxo-6(4)-oxido-(1H,5H)-pyrimidiniumbetain;
1,3-Dimethyl-5-[4-biphenylylcarbamoyl]-4(6)-oxo-6(4)-oxido-(1H,5H)-pyrimidiniumbetain;
1,3-Dimethyl-5-[4-(4-trifluormethylphenoxy)phenylcarbamoyl]-4(6)-oxo-6(4)-oxido-(1H,5H)-pyrimidiniumbetain.

Weitere besonders bevorzugte Vertreter der Formel I sind:
1,3-Dimethyl-5-[phenylcarbamoyl]-4(6)-oxo-6(4)-oxido-(1H,5H) -pyrimidiniumbetain und
1,3-Dimethyl-5-[2-fluorphenylcarbamoyl]-4(6)-oxo-6(4)-oxido-(1H,5H)-pyrimidiniumbetain.

Des weiteren ist folgende Gruppe bevorzugt:

e) Verbindungen der Formel Ia, worin $R_1$ und $R_2$ jeweils für Methyl stehen und $R_3$ für unsubstituiertes oder durch Fluor oder Chlor mono- oder disubstituiertes Phenyl oder für (Trifluormethylphenoxy)-phenyl steht.

Besonders bevorzugte Vertreter der Formel Ia sind:
1,3-Dimethyl-5-[3,4-dichlorphenylcarbamoyl]-4,6-(1H,3H,5H)-pyrimidindion;
1,3-Dimethyl-5-[2,4-difluorphenylcarbamoyl]-4,6-(1H,3H,5H)-pyrimidindion;
1,3-Dimethyl-5-[2-fluorphenylcarbamoyl]-4,6-(1H,3H,5H) -pyrimidindion;
1,3-Dimethyl-5-[3,4-dichlorphenylcarbamoyl]-4,6-(1H,3H,5H)-pyrimidindion;
1,3-Dimethyl-5-[4-biphenylylcarbamoyl]-4,6-(1H,3H,5H)-pyrimidindion;
1,3-Dimethyl-5-[4-(4-trifluormethylphenoxy)phenylcarbamoyl]-4,6-(1H,3H,5H)-pyrimidindion.

Eine weitere bevorzugte Verbindung der Formel Ia ist 1,3-Dimethyl-5-[phenylcarbamoyl]-4,6-(1H,3H,5H)-pyrimidindion.

Die Verbindungen der Formeln I und Ia werden erfindungsgemäss dadurch hergestellt, dass man eine Verbindung der Formel II

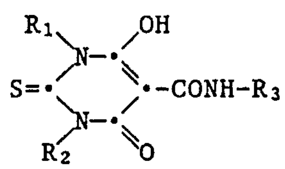

(II)

worin $R_1$, $R_2$ und $R_3$ die unter den Formeln I und Ia angegebenen Bedeutungen haben entschwefelt. Die Entschwefelung kann z.B. durch Hydrierung, vorzugsweise durch katalytische Hydrierung, vorgenommen werden. In einer bevorzugten Ausführungsform wird die Verbindung der Formel II in einem reaktionsinerten Lösungsmittel oder Lösungsmittelgemisch bei Temperaturen im Bereich von 20 bis 180° C, vorzugsweise 60 bis 120° C, insbesondere bei Rückflusstemperatur, hydriert, wobei als Hydrierungsmittel beispielsweise Trialkylzinnhydrid, Trialkylzinnhalogenid, Trialkylgermaniumhydrid, Trialkylgermaniumhalogenid, Alkylquecksilberhydrid oder Alkylquecksilberhalogenid in Betracht kommen. Bei der Verwendung eines Halogenids als Hydrierungsmittel wird zusätzlich in Gegenwart von $NaBH_4$ gearbeitet. Bei den vorstehend angegebenen Hydrierungsmitteln bedeutet Alkyl vorzugsweise $C_1$-$C_6$-Alkyl, insbesondere $C_2$-$C_4$-Alkyl, Halogenid bedeutet insbesondere Chlorid oder Bromid. Besonders geeignet sind die Chloride.

Als weiteres geeignetes Hydrierungsmittel ist Tris(trimethylsilyl)silan ($[(CH_3)_3Si]_3SiH$) zu nennen.

Die Hydride und Halogenide werden in mindestens äquimolarer Menge, bezogen auf die Ausgangsverbindung der Formel II, eingesetzt. $NaBH_4$ kann ebenfalls äquimolar zugesetzt werden.

Man kann zusätzlich in Anwesenheit eines Radikalinitiators arbeiten, wobei dieser in katalytischen Mengen zugesetzt werden kann. Geeignete Radikalinitiatoren sind z.B. 2,2′-Azobisisobutyronitril (AIBN), Peroxide wie Benzoylperoxid, aber auch UV-Licht oder Wärme. Als geeignete reaktionsinerte Lösungsmittel, allein oder im Gemisch untereinander, kommen z.B. in Frage: Aliphatische und aromatische Kohlenwasserstoffe, wie z.B. Pentan, Hexan, Petrolether, Ligroin, Benzol, Toluol, Xylole, usw.; Ether und etherartige Substanzen wie Tetrahydrofuran, Anisol, Dioxan, usw.; halogenierte Kohlenwasserstoffe wie Tetrachlorkohlenstoff, Tetrachlorethylen, Chlorbenzol, etc.

Die Verbindungen der Formeln I und Ia fallen im allgemeinen nebeneinander an, wobei man durch geeignete Wahl des Lösungsmittels das System so wählen kann, dass z.B. die schwerlöslichen Betaine der Formel I ausfallen und aus dem Reaktionsgemisch z.B. durch Filtration, entfernt werden können, während die löslicheren Verbindungen der Formel Ia aus der Lösung, z.B. durch Einengen oder Ausfällen, erhältlich sind. Man kann aber auch ein Gemisch von Verbindungen der Formeln I und Ia isolieren und anschliessend, z.B. auf Grund ihres unterschiedlichen Lösungsverhaltens, eine Trennung vornehmen, z.B. durch fraktionierte Kristallisation oder durch Säulenchromatographie. Die geschickte Wahl der Lösungsmittel führt aber unter Umständen auch zu reinen Produkten, d.h. entweder vollständig zu Verbindungen der Formel I oder zu Verbindungen der Formel Ia. Die Verbindungen der Formel I lassen sich nach üblichen Methoden durch Hydrierung, z.B. mit Raney-Nickel, $NaBH_4$, Tributylzinnhydrid, etc., in die Verbindungen der Formel Ia überführen.

Wie bereits eingangs erwähnt, können die Verbindungen der Formeln I und Ia auch als Addukte mit Basen oder Säuren vorliegen.

Während Säureadditionssalze bereits eingangs eingehend beschrieben worden sind, sollen hier noch die anorganischen und organischen Basen erwähnt werden, die als Adduktbildner in Frage kommen. Dies sind z.B. vorzugs weise tertiäre Amine wie Trialkylamine (z.B. Trimethylamin, Triethylamin oder Tripropylamin), Pyridin und Pyridinbasen (z.B. 4-Dimethylaminopyridin oder 4-Pyrrolidylaminopyridin), Picoline und Lutidine sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $K_2CO_3$ oder $Na_2CO_3$), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriummethylat. Die Base wird vorteilhafterweise in bezug auf die Reaktanden in 10 bis 100 % der äquimolaren Menge zugesetzt.

In manchen Fällen kann es von Vorteil sein, die Reaktion unter Schutzgasatmosphäre durchzuführen. Geeignete Schutzgase sind z.B. Stickstoff, Helium oder Argon.

Die Umsetzung von Verbindungen der Formel II mit Trialkylzinnhydrid, vor allem in Gegenwart eines Radikalinitiators, ist besonders bevorzugt.

Die Thiobarbiturate der Formel II sind z.B. aus der Europäischen Offenlegungsschrift EP-167,491 oder der Deutschen Offenlegungsschrift DE-2,405,732 bekannt oder können analog zu den dort beschriebenen Vertretern hergestellt werden.

Die erfindungsgemässen Wirkstoffe der Formeln I und Ia können in unterschiedlichen tautomeren Formen vorliegen, nämlich in der Keto- oder Enolform oder in einem Gemisch aus Keto- und Enolform. Die vorliegende Erfindung betrifft sowohl die einzelnen Tautomeren als auch deren Gemische, aber auch die Salze jeder dieser Formen und deren Herstellung.

Das beschriebene Herstellungsverfahren einschliesslich aller Varianten ist ein Bestandteil vorliegender Erfindung.

Wie allgemein bekannt, verursachen unter den bei Warmblütern vorkommenden Endoparasiten namentlich die Helminthen bei den von ihnen heimgesuchten Tieren grosse Schäden. Solche durch Helminthiasen verursachten Schäden können bei chronischem und vor allem bei epidemischem Auftreten der Wurmerkrankungen in Viehherden ein volkswirtschaftlich ins Gewicht fallendes Ausmass annehmen. Sie äussern sich bei den erkrankten Tieren unter anderem in Produktivitäts-Einbussen, geschwächter Widerstandskraft gegenüber weiteren Krankheiten und erhöhter Mortalität. Besonders gefährliche Wurmkrankheiten werden durch im Magen-Darmtrakt und anderen Organen parasitierende Helminthen hervorgerufen und können beispielsweise bei Wiederkäuern wie Rindern, Schafen und Ziegen sowie Pferden, Schweinen, Geflügel, Rotwild, Hunden und Katzen auftreten.

In der vorliegenden Beschreibung werden unter dem Begriff Helminthen insbesondere parasitische Würmer verstanden, die zu den Plathelminthes (Cestoden, Trematoden) und Nemathelminthes (Nematoden und Verwandte) gehören, also Bandwürmer, Saugwürmer und Rundwürmer des Gastrointestinal-Traktes und anderer Organe (z.B. Leber, Lunge, Niere, Lymphgefässe, Blut etc.).

Es ist deshalb eine vordringliche Aufgabe, therapeutische Mittel zu entwickeln, die sich zur Bekämpfung von Helminthen in allen ihren Entwicklungsstadien sowie zur Vorbeugung gegen den Befall durch diese Parasiten eignen.

Es sind zwar eine Reihe von Stoffen mit anthelmintischer Wirkung bekannt, die für die Bekämpfung der verschiedenen Helminthen-Species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene "Albendazol" (British Pat. No. 1464326; Am. J. Vet. Res. 38 , 1425-1426 (1977); Am. J. Vet. Res. 37 , 1515-1516 (1976); Am. J. Vet. Res. 38 , 807-808 (1977); Am. J. Vet. Res. 38 , 1247-1248 (1977)) besitzt nur ein begrenztes anthelmintisches Wirkungsspektrum bei Wiederkäuern. Seine Wirkung gegen Benzimidazol-resistente Nematoden und adulte Leberegel ist völlig unzureichend, wobei vor allem die pathogen wichtigen unreifen Wanderformen der letzteren bei den für das Wirtstier verträglichen Dosierungen nicht angegriffen werden.

Es wurde überraschenderweise gefunden, dass die neuen Verbindungen der Formeln I und Ia ein breites Wirkungsspektrum gegen im Tierorganismus, vor allem in Säugetieren, parasitierende Helminthen

wie Nematoden, Cestoden und Trematoden besitzen, wobei ihre Wirkung sich vorzugsweise gegen Nematoden (Rundwürmer) richtet.

Als besonderes Merkmal der Verbindungen der Formeln I und Ia ist ihre gegenüber Warmblütern überraschend hohe Verträglichkeit hervorzuheben, wodurch sie den bekannten Thiobarbitursäurederivaten überlegen sind. Ihre praktische Handhabung bei der Behandlung wurmerkrankter Tiere wird dadurch ausserordentlich erleichtert, da sie auch in höheren Dosen von den medikierten Tieren symptomlos vertragen werden.

Die erfindungsgemässen neuen Wirkstoffe der Formeln I und Ia eignen sich beispielsweise zur Bekämpfung parasitärer Nematoden der Ordnungen (nach K.I. Skrajabin)
Rhabditida
Ascaridida
Spirurida
Trichocephalida
oder zur Bekämpfung von Cestoden der Ordnungen (nach Wardle & McLeod)
Cyclophyllidae
Pseudophyllidae
oder zur Bekämpfung von Trematoden der Ordnung
Digenea
bei Haus- und Nutztieren wie Rindern, Schafen, Ziegen, Pferden, Schweinen, Rotwild, Katzen, Hunden und Geflügel. Sie können den Tieren sowohl als Einzeldosis als auch wiederholt verabreicht werden, wobei die einzelnen Gaben je nach Tierart vorzugsweise zwischen 1 und 20 mg pro kg Körpergewicht betragen. Eine protrahierte Verabreichung führt in manchen Fällen zu einer besseren Wirkung oder gestattet die Anwendung geringerer Gesamtdosen im Vergleich mit anderen Behandlungsmethoden.

Die erfindungsgemäsen Mittel werden hergestellt, indem die Wirkstoffe der Formeln I oder Ia mit flüssigen und/oder festen Formulierungshilfsstoffen durch schrittweises Vermischen und/oder Vermahlen derart in Kontakt gebracht werden, dass eine anwendungskonforme optimale Entfaltung der anthelmintischen Aktivität der Formulierung erzielt wird.

Die Formulierungsschritte können durch Kneten, Granulieren (Granulate) und gegebenenfalls Pressen (Pellets) ergänzt werden.

Als Formulierungshilfsstoffe dienen beispielsweise feste Trägerstoffe, Lösungsmittel und gegebenenfalls oberflächenaktive Stoffe (Tenside).

Zur Bereitung der erfindungsgemässen Mittel lassen sich folgende Formulierungshilfsstoffe verwenden: Feste Trägerstoffe wie z.B. Kaolin, Talkum, Bentonit, Kochsalz, Calciumphosphat, Kohlenhydrate, Cellulosepulver, Baumwollsaatmehl, Polyäthylenglykoläther, gegebenenfalls Bindemittel wie z.B. Gelatine, lösliche Cellulosederivate, gewünschtenfalls unter Zusatz von oberflächenaktiven Stoffen wie ionischen oder nichtionischen Dispersionsmitteln; ferner natürliche Gesteinsmehle wie Calcit, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Ether und Ester, wie z.B. Ethanol, Ethylenglykol, Ethylenglykolmonomethyl- oder -ethylether, Ketone wie z.B. Cyclohexanon, stark polare Lösungsmittel wie z.B. N-Methyl-2-pyrrolidon, Dimethylsulfoxid oder Dimethylformamid, sowie gegebenenfalls epoxydierte Pflanzenöle, wie z.B. epoxydiertes Kokosnussöl oder Sojaöl, und Wasser.

Als oberflächenaktive Verbindungen kommen je nach Art des zu formulierenden Wirkstoffes der Formeln I oder Ia nicht-ionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können, genannt. Ferner sind auch die Fettsäure-methyltaurinsalze zu erwähnen.

Häufig werden sog. synthetische Tenside verwendet, insbesondere Fettsulfonate, Fettsulfate, sulfonierte

Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes oder Phospholipide in Frage.

Als nicht-ionische Tenside kommen in erster Linie Polyglykoletherderivate von aliphatischen oder cycloaliphatischen Alkoholen, gesättigten oder ungesättigten Fettsäuren und Alkyphenolen in Frage, die 3 bis 30 Glykolethergruppen und 8 bis 20 Kohlenstoffatome im aliphatischen Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-einheit 1 bis 5 Ethylenglykoleinheiten.

Als Beispiele nicht-ionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual",
MC Publishing Corp., Ridgewood New Jersey, 1980;
Sisley and Wood, "Encyclopedia of Surface Active Agents",
Chemical Publishing Co., Inc. New York, 1980.

Als Bindemittel für Tabletten und Boli kommen chemisch abgewandelte, in Wasser oder Alkohol lösliche, polymere Naturstoffe in Frage, wie Stärke-, Cellulose- oder Protein-derivate (z.B. Methylcellulose, Carboxymethylcellulose, Ethylhydroxyethylcellulose, Proteine wie Zein, Gelatine und dergleichen) sowie synthetische Polymere wie z.B. Polyvinylalkohol, Polyvinylpyrrolidon etc.. Ferner sind in Tabletten Füllstoffe (z.B. Stärke, mikrokristalline Cellulose, Zucker, Milchzucker etc.), Gleitmittel und Sprengmittel enthalten.

Liegen die anthelmintischen Mittel in Form von Futterkonzentraten vor, so dienen als Trägerstoffe z.B. Leistungsfutter, Futtergetreide oder Proteinkonzentrate. Solche Futterkonzentrate oder -mittel können ausser den Wirkstoffen noch Zusatzstoffe, Vitamine, Antibiotika, Chemotherapeutika oder Pestizide, vornehmlich Bakteriostatika, Fungistatika, Coccidiostatika, oder auch Hormonpräparate, Stoffe mit anaboler Wirkung oder das Wachstum begünstigende, die Fleischqualität von Schlachttieren beeinflussende oder in anderer Weise für den Organismus nützliche Stoffe enthalten. Werden die Mittel oder die darin enthaltenen Wirkstoffe der Formeln I oder Ia direkt dem Futter oder den Viehtränken zugesetzt, so enthält das Fertigfutter oder die Fertigtränke die Wirkstoffe vorzugsweise in einer Konzentration von etwa 0,0005 bis 0,02 Gewichtsprozent (5-200 ppm).

Die Applikation der erfindungsgemässen Mittel an die zu behandelnden Tiere kann peroral, parenteral oder subcutan durchgeführt werden, wobei die Mittel in Form von Lösungen, Emulsionen, Suspensionen (Drenchs), Pulvern, Tabletten, Boli und Kapseln oder als Granulat vorliegen.

Die erfindungsgemässen anthelmintischen Mittel enthalten in der Regel 0,1 bis 99 Gew.-%, insbesondere 0,1 bis 95 Gew.-% Wirkstoff der Formel I, der Formel Ia oder eines Gemisches von beiden, 99,9 bis 1 Gew.-%, insbesondere 99,8 bis 5 Gew.-%, eines festen oder flüssigen Zusatzstoffes, darunter 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Derartige vom Endverbraucher verwendete anthelmintische Mittel sind ebenfalls ein Bestandteil der vorliegenden Erfindung.

In jedem der erfindungsgemässen Verfahren zur Schädlingsbekämpfung bzw. der erfindungsgemässen Schädlingsbekämpfungsmittel können die Wirkstoffe der Formeln I und Ia in allen tautomeren Formen, deren Mischungen oder in Form ihrer Salze eingesetzt werden.

Die Erfindung schliesst auch ein Verfahren zum prophylaktischen Schutz von Tieren gegen parasitäre Helminthen ein, das dadurch gekennzeichnet ist, dass man die Wirkstoffe der Formeln I und Ia bzw. die Wirkstofformulierungen als Zusatz zum Futter oder zu den Tränken oder auch in fester oder flüssiger Form oral, durch Injektion oder parenteral den Tieren appliziert.

Die nachfolgenden Beispiele dienen der Illustration der Erfindung, ohne sie einzuschränken.

## 1. Herstellungsbeispiele

### 1.1 Herstellung von 1,3-Dimethyl-5-[3,4-dichlorphenylcarbamoyl]-4(6)-oxo-6(4)-oxido-(1H,5H)-pyrimidiniumbetain

Eine Lösung von 15,5 g 1,3-Dimethyl-5-[3,4-dichlorphenylcarbamoyl]-2-thiobarbitursäure, 0,54 g 2,2'-Azobisisobutyronitril und 37,54 g Tributylzinnhydrid werden 3,5 Stunden in Benzol und unter Stickstoffatmosphäre unter Rückfluss und unter Rühren erhitzt. Nach Abkühlen des Reaktionsgemisches auf Raumtemperatur wird das ausgefallene, kristalline Produkt abfiltriert, mehrfach mit Hexan gewaschen und getrocknet. Man erhält 3,5 g der Titelsubstanz. Smp. 292-305 °C.
$^1$H-NMR (300 MHz, DMSO $d_6$, TMS): 11.21, s, NH; 9.38, s, H-C (3); 8.13, d, J=2, H-C (2'); 7.52, d, J=9, H-C (5'); 7.44, dxd, J= 9 und 2, H-C (6'); 3.38, s, $2xCH_3$.

### 1.2 Herstellung von 1,3-Dimethyl-5-[3,4-dichlorphenylcarbamoyl]-4,6-(1H,3H,5H)-pyrimidindion

Das gemäss Beispiel 1.1 gewonnene Filtrat wird eingeengt und das ausgefallene Produkt abfiltriert und mehrfach aus Ethanol umkristallisiert. Man erhält 7,6 g der Titelsubstanz, mit Smp. 174-176 °C.
$^1$H-NMR (300 MHz, $CDCl_3$, TMS): 18.25, s, OH; 11.91, s, NH; 7.74, d, J=2, H-C (2'); 7.36, d, J=9), H-C (5'); 7.22, dxd, J=9 und 2, H-C (6'); 4.50, s, $H_2$-C(3); 3.02, s, $CH_3$; 2.95, s, $CH_3$.

### 1.3 Herstellung von 1,3-Dimethyl-5-[4-chlorphenylcarbamoyl]-4,6-(1H,3H,5H)-pyrimidindion

a) Eine Lösung von 0,2 g 1,3-Dimethyl-5-[4-chlorphenylcarbamoyl]-2-thiobarbitursäure, 7,7 mg Azoisobutyronitril und 894 mg Tributylzinnhydrid werden in 14 ml Toluol 20 Stunden unter Rückfluss gerührt, wobei man unter Stickstoffatmosphäre arbeitet. Das auf Raumtemperatur abgekühlte Gemisch wird eingeengt und das auskristallisierte Rohprodukt abfiltriert, mit Hexan gewaschen, getrocknet und aus Ethanol/Wasser umkristallisiert. Man erhält 132 mg der Titelsubstanz mit Smp. 162-163 °C.

b) Eine Suspension von 200 mg 1,3-Dimethyl-5-[4-chlorphenylcarbamoyl]-4(6)-oxo-6(4)-oxido-(1H,5H)-pyrimidiniumbetain und 396 mg Tributylzinnhydrid in Benzol werden unter Argon 72 Stunden unter Rückfluss gerührt. Das auf Raumtemperatur abgekühlte Reaktionsgemisch wird eingeengt und das auskristallisierte Rohprodukt abfiltriert, mit Petrolether gewaschen, getrocknet und aus Ethanol/Wasser umkristallisiert. Man erhält 85 mg der Titelsubstanz mit Smp. 162-163 °C.

Die nach a und b hergestellte Substanz zeigt folgendes 1H-NMR-Spektrum (300 MHz, $CDCl_3$, TMS): 18.20, s, OH; 11.85, s, NH; 7.43, d, J=9.5, 2H; 7.28, d, J=9.5, 2H; 4.48, s, $H_2$-C(3); 3.00, s, 3H; 2.95, s, 3H.

Analog zu den beschriebenen Vorgehensweisen lassen sich auch die in den folgenden Tabellen genannten Vertreter der Formeln I und Ia herstellen.

In den folgenden Tabellen steht $C_6H_5$ für einen unsubstituierten Phenylrest, $C_6H_4$ für einen monosubstituierten Phenylrest (mit anschliessender Definition des Substituenten), und $C_6H_3$ und $C_6H_2$ stehen für di- und trisubstituierte Phenylreste (mit anschliessender Definition der Substituenten).

Tabelle 1: Verbindungen der Formel I

| Verb. Nr. | $R_1$ | $R_2$ | $R_3$ | physikalische Daten Smp. [°C] $^1H$-NMR (300 MHz, DMSO $d_6$, TMS) |
|---|---|---|---|---|
| 1.1 | $CH_3$ | $CH_3$ | $C_6H_5$ | 11.00. s, NH; 9.37, s, H-C(3); 3.38, s, $2xCH_3$. |
| 1.2 | $CH_3$ | $CH_3$ | $C_6H_4$(2-Cl) | 11.72, s, NH; 9.41, s, H-C(3); 3.40, s, $2xCH_3$. |
| 1.3 | $CH_3$ | $CH_3$ | $C_6H_4$(4-Cl) | 295-300° |
| 1.4 | $CH_3$ | $CH_3$ | $C_6H_3$(3,4-$Cl_2$) | 11.21, s, NH; 9.38, s, H-C(3); 3.38, s, $2xCH_3$. |
| 1.5 | $CH_3$ | $CH_3$ | $C_6H_4$(3-Br) | |
| 1.6 | $CH_3$ | $CH_3$ | $C_6H_4$(2-F) | 11.60, s, NH; 9.40, s, H-C(3); 3.38, s, $2xCH_3$. |
| 1.7 | $CH_3$ | $CH_3$ | $C_6H_3$(2,4-$F_2$) | 11.56, s, NH; 9.41, s, H-C(3); 3.39, s, $2xCH_3$. |
| 1.8 | $CH_3$ | $CH_3$ | $C_6H_4$(2-$CH_3$) | 11.10, s, NH; 9.38, s, H-C(3); 3.39, s, $2xCH_3$. |
| 1.9 | $C_2H_5$ | $C_2H_5$ | $C_6H_3$(3,4-$Cl_2$) | |
| 1.10 | $CH_3$ | $CH_3$ | $C_6H_4$(4-$C_6H_5$) | 11.19, s, NH; 9.37, s, H-C(3); 3.39, s, $2xCH_3$. |
| 1.11 | $CH_3$ | $CH_3$ | $C_6H_3$(2-$OCH_3$)(5-$C_6H_5$) | 11.51, s, NH; 9.38, s, H-C(3); 3.39, s, $2xCH_3$. |
| 1.12 | $CH_3$ | $CH_3$ | $C_6H_4$(2-$OCH_3$) | |

Tabelle 1: Fortsetzung

| Verb. Nr. | $R_1$ | $R_2$ | $R_3$ | physikalische Daten Smp. [°C] $^1$H-NMR (300 MHz, DMSO $d_6$, TMS) |
|---|---|---|---|---|
| 1.13 | $CH_3$ | $CH_3$ | $C_6H_4[4\text{-}OC_6H_4(4'\text{-}CF_3)]$ | 233-238° |
| 1.14 | $CH_3$ | $CH_3$ | $C_6H_4[4\text{-}OC_6H_4(3'\text{-}OCF_3)]$ | |
| 1.15 | $CH_3$ | $CH_3$ | $C_6H_4[4\text{-}OC_6H_4(3'\text{-}CF_3)]$ | |
| 1.16 | $CH_3$ | $CH_3$ | $C_6H_3(2\text{-}Cl)[4\text{-}CH(CH_3)COOCH_3]$ | |
| 1.17 | $C_2H_5$ | $CH_3$ | $C_6H_4[4\text{-}OC_6H_4(4'\text{-}CF_3)]$ | |
| 1.18 | $C_2H_5$ | $CH_3$ | $C_6H_4(4\text{-}Cl)$ | |
| 1.19 | $C_2H_5$ | $C_2H_5$ | $C_6H_3(2\text{-}Cl,\ 4\text{-}F)$ | |
| 1.20 | $CH_2CH{=}CH_2$ | $CH_3$ | $C_6H_4[4\text{-}OC_6H_4(4'\text{-}CF_3)]$ | |
| 1.21 | $CH_2CH{=}CH_2$ | $CH_3$ | $C_6H_4[4\text{-}OC_6H_4(3'\text{-}CF_3)]$ | |
| 1.22 | Cyclopropyl | $CH_3$ | $C_6H_4[4\text{-}OC_6H_4(3'\text{-}CF_3)]$ | |
| 1.23 | Cyclohexyl | $CH_3$ | $C_6H_4(4\text{-}Cl)$ | |
| 1.24 | Cyclopropyl | Cyclopropyl | $C_6H_4[4\text{-}OC_6H_4(3'\text{-}CF_3)]$ | |
| 1.25 | $CH_3$ | $CH_3$ | $C_6H_2[2,6\text{-}(CH_3)_2][4\text{-}OC_6H_4(4'\text{-}CF_3)]$ | |
| 1.26 | $CH_3$ | $CH_3$ | $C_6H_3(2\text{-}iso\text{-}C_3H_7)[4\text{-}OC_6H_4(4'\text{-}CF_3)]$ | |
| 1.27 | $CH_3$ | $CH_3$ | $C_6H_4[4\text{-}OC_6H_4(3'\text{-}CH_2CN)]$ | |
| 1.28 | $CH_3$ | $CH_3$ | $C_6H_3(3\text{-}Cl)[4\text{-}OC_6H_3(3'\text{-}Cl,4'\text{-}CF_3)]$ | |
| 1.29 | $CH_3$ | $CH_3$ | $C_6H_3(2,4\text{-}F_2)$ | • HCl |

Tabelle 1: Fortsetzung

| Verb. Nr. | $R_1$ | $R_2$ | $R_3$ | physikalische Daten Smp. [°C] $^1$H-NMR (300 MHz, DMSO $d_6$, TMS) |
|---|---|---|---|---|
| 1.30 | $CH_3$ | $C_2H_5$ | $C_6H_4(2-F)$ | 296-298° |
| 1.31 | $CH_3$ | $C_2H_5$ | $C_6H_3(2,4-F_2)$ | 308-310° |
| 1.32 | $CH_3$ | $CH_2-CH=CH_2$ | $C_6H_4(2-F)$ | 219-221° |
| 1.33 | $CH_3$ | $CH_2-CH=CH_2$ | $C_6H_3(2,4-F_2)$ | 252-254° |
| 1.34 | $CH_3$ | $CH_3$ | $C_6H_2[3,4,5-(OCH_3)_3]$ | |
| 1.35 | $CH_3$ | $CH_3$ | $C_6H_3(2-CH_3)[4-OC_6H_4(4'CF_3)]$ | |
| 1.36 | $CH_3$ | $CH_3$ | $C_6H_4[4-OC_6H_4(4'-OCH_3)]$ | |
| 1.37 | $CH_3$ | $CH_3$ | $C_6H_4[4-OC_6H_4(4'-OCF_3)]$ | |
| 1.38 | $CH_3$ | $CH_3$ | $C_6H_3(2-OC_6H_5)(4-Cl)$ | |
| 1.39 | $C_6H_5$ | $CH_3$ | $C_6H_5$ | |

Tabelle 2: Verbindungen der Formel Ia

| Verb. Nr. | $R_1$ | $R_2$ | $R_3$ | physikalische Daten Smp. [°C] |
|---|---|---|---|---|
| 2.1 | $CH_3$ | $CH_3$ | $C_6H_5$ | 119-121 |
| 2.2 | $CH_3$ | $CH_3$ | $C_6H_4(2\text{-}Cl)$ | 153-155 |
| 2.3 | $CH_3$ | $CH_3$ | $C_6H_4(4\text{-}Cl)$ | 162-163 |
| 2.4 | $CH_3$ | $CH_3$ | $C_6H_3(3,4\text{-}Cl_2)$ | 174-176 |
| 2.5 | $CH_3$ | $CH_3$ | $C_6H_4(3\text{-}Br)$ | |
| 2.6 | $CH_3$ | $CH_3$ | $C_6H_4(2\text{-}F)$ | 138-140 |
| 2.7 | $CH_3$ | $CH_3$ | $C_6H_3(2,4\text{-}F_2)$ | 225-226 |
| 2.8 | $CH_3$ | $CH_3$ | $C_6H_4(2\text{-}CH_3)$ | 126-128 |
| 2.9 | $C_2H_5$ | $C_2H_5$ | $C_6H_3(3,4\text{-}Cl_2)$ | |
| 2.10 | $CH_3$ | $CH_3$ | $C_6H_4(4\text{-}C_6H_5)$ | 155-158 |
| 2.11 | $CH_3$ | $CH_3$ | $C_6H_3(2\text{-}OCH_3)(5\text{-}C_6H_5)$ | 165-167 |
| 2.12 | $CH_3$ | $CH_3$ | $C_6H_4(2\text{-}OCH_3)$ | 145-146 |
| 2.13 | $CH_3$ | $CH_3$ | $C_6H_4[4\text{-}OC_6H_4(4'\text{-}CF_3)]$ | 106-109 |
| 2.14 | $CH_3$ | $CH_3$ | $C_6H_4[4\text{-}OC_6H_4(3'\text{-}OCF_3)]$ | |
| 2.15 | $CH_3$ | $CH_3$ | $C_6H_4[4\text{-}OC_6H_4(3'\text{-}CF_3)]$ | |
| 2.16 | $CH_3$ | $CH_3$ | $C_6H_3(2\text{-}Cl)[4\text{-}CH(CH_3)COOCH_3]$ | |
| 2.17 | $C_2H_5$ | $CH_3$ | $C_6H_4[4\text{-}OC_6H_4(4'\text{-}CF_3)]$ | 102-103 |
| 2.18 | $C_2H_5$ | $CH_3$ | $C_6H_4(4\text{-}Cl)$ | |

Tabelle 2: Fortsetzung

| Verb. Nr. | $R_1$ | $R_2$ | $R_3$ | physikalische Daten Smp. [°C] |
|---|---|---|---|---|
| 2.19 | $C_2H_5$ | $C_2H_5$ | $C_6H_3$(2-Cl, 4-F) | |
| 2.20 | $CH_2CH=CH_2$ | $CH_3$ | $C_6H_4$[4-$OC_6H_4$(4'-$CF_3$)] | 88-90 |
| 2.21 | $CH_2CH=CH_2$ | $CH_3$ | $C_6H_4$[4-$OC_6H_4$(3'-$CF_3$)] | |
| 2.22 | Cyclopropyl | $CH_3$ | $C_6H_4$[4-$OC_6H_4$(3'-$CF_3$)] | |
| 2.23 | Cyclohexyl | $CH_3$ | $C_6H_4$(4-Cl) | |
| 2.24 | Cyclopropyl | Cyclopropyl | $C_6H_4$[4-$OC_6H_4$(3'-$CF_3$)] | |
| 2.25 | $CH_3$ | $CH_3$ | $C_6H_2$[2,6-$(CH_3)_2$][4-$OC_6H_4$(4'-$CF_3$)] | |
| 2.26 | $CH_3$ | $CH_3$ | $C_6H_3$(2-iso-$C_3H_7$)[4-$OC_6H_4$(4'-$CF_3$)] | |
| 2.27 | $CH_3$ | $CH_3$ | $C_6H_4$[4-$OC_6H_4$(3'-$CH_2CN$)] | |
| 2.28 | $CH_3$ | $CH_3$ | $C_6H_3$(3-Cl)[4-$OC_6H_3$(3'-Cl,4'-$CF_3$)] | |
| 2.29 | $CH_3$ | $CH_3$ | $C_6H_3$(2,4-$F_2$) | • HCl |

Tabelle 2: Fortsetzung

| Verb. Nr. | $R_1$ | $R_2$ | $R_3$ | physikalische Daten Smp. [°C] |
|---|---|---|---|---|
| 2.30 | $CH_3$ | $C_2H_5$ | $C_6H_4(2\text{-}F)$ | 121-123 |
| 2.31 | $CH_3$ | $C_2H_5$ | $C_6H_3(2,4\text{-}F_2)$ | 98-100 |
| 2.32 | $CH_3$ | $CH_2\text{-}CH\text{=}CH_2$ | $C_6H_4(2\text{-}F)$ | 78-81 |
| 2.33 | $CH_3$ | $CH_2\text{-}CH\text{=}CH_2$ | $C_6H_3(2,4\text{-}F_2)$ | 68-70 |
| 2.34 | $CH_3$ | $C_2H_5$ | $C_6H_3(2\text{-}Cl)[4\text{-}CH(CH_3)\text{-}COOCH_3]$ | |
| 2.35 | $CH_3$ | $CH_2\text{-}CH\text{=}CH_2$ | $C_6H_3(2\text{-}Cl)[4\text{-}CH(CH_3)\text{-}COOCH_3]$ | |
| 2.36 | $CH_3$ | $CH_3$ | $C_6H_2[3,4,5\text{-}(OCH_3)_3]$ | |
| 2.37 | $CH_3$ | $CH_3$ | $C_6H_3(2\text{-}CH_3)[4\text{-}OC_6H_4(4'\text{-}CF_3)]$ | |
| 2.38 | $CH_3$ | $CH_3$ | $C_6H_4[4\text{-}OC_6H_4(4'\text{-}OCH_3)]$ | |
| 2.39 | $CH_3$ | $CH_3$ | $C_6H_4[4\text{-}OC_6H_4(4'\text{-}OCF_3)]$ | |
| 2.40 | $CH_3$ | $CH_3$ | $C_6H_3(2\text{-}OC_6H_5)(4\text{-}Cl)$ | |
| 2.41 | $C_6H_5$ | $CH_3$ | $C_6H_5$ | |

2. Formulierungsbeispiele (% = Gewichtsprozent)

| 2.1. Emulsions-Konzentrate | a) | b) | c) |
|---|---|---|---|
| Wirkstoff aus Tabelle 1 oder 2 | 25 % | 40% | 50% |
| Ca-Dodecylbenzolsulfonat | 5 % | 8% | 6% |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus diesen Konzentraten können durch Verdünnen mit Wasser Emulsionen jeder gewünschten Konzentration hergestellt werden.

| 2.2. Emulsions-Konzentrate | a) | b) | c) |
|---|---|---|---|
| Wirkstoff aus Tabelle 1 oder 2 | 10 % | 8 % | 60 % |
| Octylphenolpolyethylenglykolether (4-5 Mol Ethylenoxid) | 3 % | 3 % | 2 % |
| Ca-Dodecylbenzolsulfonat | 3 % | 4 % | 4 % |
| Ricinusölpolyethylenglykolether (35 Mol Ethylenoxid) | 4 % | 5 % | 4 % |
| Cyclohexanon | 30 % | 40 % | 15 % |
| Xylolgemisch | 50 % | 40 % | 15 % |

Aus diesen Konzentraten können durch Verdünnen mit Wasser Emulsionen jeder gewünschten Konzentration hergestellt werden.

| 2.3. Suspensions-Konzentrat | |
|---|---|
| Wirkstoff aus Tabelle 1 oder 2 | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| 2.4. In Wasser dispergierbare Pulver | a) | b) | c) |
|---|---|---|---|
| Wirkstoff aus Tabelle 1 oder 2 | 25 % | 50 % | 75 % |
| Na-Ligninsulfonat | 5 % | 5 % | - |
| Oelsäure | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.5. Pulver | a) | b) |
|---|---|---|
| Wirkstoff der Tabelle 1 oder 2 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff und Vermahlen des Gemisches erhält man gebrauchsfertige Pulver.

| 2.6. Granulat | a) | b) |
|---|---|---|
| Wirkstoff der Tabelle 1 oder2 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft. Solche Granulate können dem Viehfutter beigemischt werden.

| 2.7. Granulat | |
|---|---|
| Wirkstoff aus Tabelle 1 oder 2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.8. Granulat | |
|---|---|
| Wirkstoff aus Tabelle 1 oder 2 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.9. Tabletten bzw. Boli | |
|---|---|
| I Wirkstoff der Tabelle 1 oder 2 | 33,00 % |
| Methylcellulose | 0,80 % |
| Kieselsäure hochdispers | 0,80 % |
| Maisstärke | 8,40 % |
| II Milchzucker krist. | 22,50 % |
| Maisstärke | 17,00 % |
| mikrokrist. Cellulose | 16,50 % |
| Magnesiumstearat | 1,00 % |

I Methylcellulose wird in Wasser eingerührt. Nachdem das Material gequollen ist, wird Kieselsäure eingerührt und das Gemisch homogen suspendiert. Wirkstoff und Maisstärke werden gemischt. In diese Mischung wird die wässrige Suspension eingearbeitet und zu einem Teig geknetet. Die so erhaltene Masse wird durch ein 12 M-Sieb granuliert und getrocknet.
II Alle 4 Hilfsstoffe werden gut gemischt.
III Die gemäss I und II erhaltenen Vormischungen werden gemischt und zu Tabletten oder Boli verpresst.

2.10. Injektabiles

| A. Oeliges Vehikel (langsame Freisetzung) | |
|---|---|
| Ein Wirkstoff aus Tabelle 1 oder 2 | 0,1-1,0 g |
| Erdnussöl | ad 100 ml |
| Ein Wirkstoff aus Tabelle 1 oder 2 | 0,1-1,0 g |
| Sesamöl | ad 100 ml |

Herstellung: Der Wirkstoff wird in einem Teil des Oels unter Rühren und gegebenenfalls leichtem Erwärmen gelöst, nach Abkühlung auf das Sollvolumen aufgefüllt und durch ein geeignetes Membranfilter mit 0,22 µm sterilfiltriert.

| B. Wassermischbare Lösungen (mittlere Freisetzungsgeschwindigkeit) | |
|---|---|
| Ein Wirkstoff aus Tabelle 1 oder 2 | 0,1-1,0 g |
| 4-Hydroxymethyl-1,3-dioxolan (Glycerol Formal) | 40 g |
| 1,2-Propandiol | ad 100 ml |
| Ein Wirkstoff aus Tabelle 1 oder 2 | 0,1-1,0 g |
| Glycerindimethylketal | 40 g |
| 1,2-Propandiol | ad 100 ml |

Herstellung: Der Wirkstoff wird in einem Teil des Lösungsmittels unter Rühren gelöst, auf das Sollvolumen aufgefüllt und durch ein geeignetes Membranfilter mit 0.22 µm sterilfiltriert.

| C. Wässriges Solubilisat (rasche Freisetzung) | |
|---|---|
| Ein Wirkstoff aus Tabelle 1 oder 2 | 0,1-1,0 g |
| Polyäthoxyliertes Ricinusöl (40 Aethylenoxideinheiten) | 10 g |
| 1,2-Propandiol | 20 g |
| Benzylalkohol | 1 g |
| Aqua ad injekt. | ad 100 ml |
| Ein Wirkstoff aus Tabelle 1 oder 2 | 0,1-1,0 g |
| Polyäthoxyliertes Sorbitanmonooleat (20 Aethylenoxideinheiten) | 8 g |
| 4-Hydroxymethyl-1,3-dioxolan (Glycerol Formal) | 20 g |
| Benzylalkohol | 1 g |
| Aqua ad injekt. | ad 100 ml |

Herstellung: Der Wirkstoff wird in den Lösungsmitteln und dem Tensid gelöst und mit Wasser auf das Sollvolumen aufgefüllt. Anschliessend erfolgt Sterilfiltration durch ein geeignetes Membranfilter mit 0,22 µm Porendurchmesser.

Die wässrigen Systeme können bevorzugterweise auch für die orale und/oder intraruminale Applikation eingesetzt werden.

## 3. Biologische Beispiele

Die anthelmintische Wirksamkeit wird anhand folgender Versuche demonstriert:

### 3.1. Versuch an mit Nematoden wie Haemonchus contortus und Trichostrongylus colubriformis infizierten Schafen

Der Wirkstoff wird in Form einer Suspension mit einer Magensonde oder durch Pansen-Injektion Schafen verabreicht, die vorher mit Nematoden wie Haemonchus contortus und Trichostrongylus colubriformis künstlich infiziert wurden. Pro Versuch resp. pro Dosis werden 1 bis 3 Tiere verwendet. Jedes Schaf wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen wird.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich.

In diesem Test wird mit Verbindungen der Formeln I und Ia eine starke Reduzierung des Nematodenbefalls erzielt. So wird beispielsweise beim Einsatz von 20 mg Wirksubstanz pro kg Körpergewicht mit den nachfolgenden Verbindungen eine Reduzierung des Nematodenbefalls von ca. 90 % bewirkt: 1.1, 1.2, 1.3, 1.4, 1.6, 1.7, 1.8, 1.10, 1.11, 1.13, 2.1, 2.2, 2.3, 2.4, 2.6, 2.7, 2.8, 2.10, 2.11, 2.12 und 2.13. Dieses Ergebnis wird bei einzelnen Verbindungen auch noch mit weiter herabgesetzter Dosis, beispielsweise mit 10 mg Wirksubstanz pro kg Körpergewicht oder noch geringeren Mengen an Wirksubstanz, erreicht.

### 3.2. Versuch an mit Cestoden wie Moniezia benedeni infizierten Schafen

Der Wirkstoff wird in Form einer Suspension mit einer Magensonde oder durch Pansen-Injektion Schafen verabreicht, die vorher mit Cestoden wie Moniezia benedeni artifiziell infiziert wurden. Pro Versuch resp. pro Dosis werden 3 Tiere verwendet. Jedes Schaf wird mit nur einer einzigen Dosis behandelt.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich. In diesem Versuch bewirken Wirkstoffe aus der Tabelle 1 oder 2, wie beispielsweise die Verbindungen Nr. 1.1, 1.2, 1.3, 1.4, 1.6, 1.7, 1.8, 1.10, 1.11, 1.13, 2.1, 2.2, 2.3, 2.4, 2.6, 2.7, 2.8, 2.10, 2.11, 2.12 und 2.13 bei Dosen von weniger als 20 mg/kg Körpergewicht eine ca. 90%ige Reduktion des Cestodenbefalls.

3.3. Versuch an mit Fasciola hepatica infizierten Schafen

Der Wirkstoff wird in Form einer Suspension mit einer Magensonde oder durch Pansen-Injektion Schafen verabreicht, die vorher mit Fasciola hepatica artifiziell infiziert wurden. Pro Versuch resp. pro Dosis werden 3 Tiere verwendet. Jedes Tier wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen wird.

Drei bis vier Wochen nach der Behandlung werden die Schafe getötet und seziert. Die Auswertung erfolgt durch das Auszählen der nach der Behandlung in den Gallengängen zurückgebliebenen Leberegel. Gleichzeitig und gleichartig infizierte aber unbehandelte Schafe dienen als Kontrolle resp. Vergleich. Der Unterschied der in den beiden Gruppen festgestellten Anzahl von Leberegeln ergibt den Wirkungsgrad des geprüften Wirkstoffs.

In diesem Versuch zeigen Wirkstoffe aus der Tabelle 1 oder 2 bei Dosen von weniger als 20 mg/kg Körpergewicht eine etwa 95%ige Wirksamkeit gegen Fasciola hepatica. Unter diesen Wirkstoffen sind die Verbindungen Nr. 1.1, 1.2, 1.3, 1.4, 1.6, 1.7, 1.8, 1.10, 1.11, 1.13, 2.1, 2.2, 2.3, 2.4, 2.6, 2.7, 2.8, 2.10, 2.11, 2.12 und 2.13 bei 12 mg/g Körpergewicht voll wirksam gegen Fasciola hepatica.

## Ansprüche

1. Verbindungen der allgemeinen Formel I

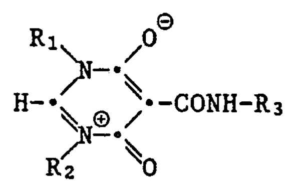

(I)

und in hydrierter Form der Formel Ia

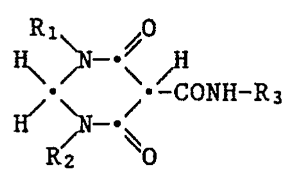

(Ia)

worin

$R_1$ und $R_2$ unabhängig voneinander für $C_1$-$C_6$-Alkyl, Allyl, $C_3$-$C_6$-Cycloalkyl oder Phenyl stehen; und $R_3$ für unsubstituiertes oder ein- oder mehrfach substituiertes Phenyl, Biphenylyl oder Phenoxyphenyl steht; wobei deren Substituenten ausgewählt sind aus der Gruppe Halogen, Cyano, $C_1$-$C_3$-Alkyl, $C_1$-$C_3$-Haloalkyl, $C_1$-$C_3$-Alkoxy, $C_1$-$C_3$-Haloalkoxy, $C_1$-$C_3$-Cyanoalkyl, Nitro, Amino oder durch $C_1$-$C_3$-Alkoxycarbonyl substituiertes $C_1$-$C_3$-Alkyl, unter Einschluss ihrer tautomeren Formen und physiologisch verträglichen Salze.

2. Verbindungen nach Anspruch 1 der Formeln I und Ia worin $R_1$ und $R_2$ unabhängig voneinander für Methyl, Ethyl, Isopropyl, Allyl oder Cyclopropyl stehen; und $R_3$ für ein- bis dreifach substituiertes Phenyl steht, wobei die Substituenten am Phenyl ausgewählt sind aus der Gruppe Fluor, Chlor, Brom, Methyl, Ethyl, Nitro, Amino, Trifluormethyl, Methoxy, Ethoxy, $CH_2CN$ oder $-CH(CH_3)COOCH_3$.

3. Verbindungen nach Anspruch 1 der Formeln I und Ia worin $R_1$ und $R_2$ unabhängig voneinander für Methyl, Ethyl, Isopropyl, Allyl oder Cyclopropyl stehen; und $R_3$ für ein- bis dreifach substituiertes Phenoxyphenyl steht, wobei die Substituenten am Phenyl ausgewählt sind aus der Gruppe Fluor, Chlor, Brom, Methyl, Ethyl, Nitro, Amino, Trifluormethyl, Methoxy, Ethoxy, $CH_2CN$ oder $-CH(CH_3)COOCH_3$.

4. Verbindungen nach Anspruch 1 der Formeln I und Ia worin $R_1$ und $R_2$ unabhängig voneinander für Methyl, Ethyl, Isopropyl, Allyl oder Cyclopropyl stehen; und $R_3$ für ein- bis dreifach substituiertes Biphenylyl steht, wobei die Substituenten am Phenyl ausgewählt sind aus der Gruppe Fluor, Chlor, Brom, Methyl, Ethyl, Nitro, Amino, Trifluormethyl, Methoxy, Ethoxy, $CH_2CN$ oder $-CH(CH_3)COOCH_3$.

5. Verbindungen nach Anspruch 1 der Formeln I und Ia, worin $R_1$ und $R_2$ unabhängig voneinander für

Methyl, Ethyl oder Allyl stehen und $R_3$ für unsubstituiertes oder durch einen oder zwei Substituenten, ausgewählt aus der Gruppe Fluor, Chlor, Methyl und Methoxy, substituiertes Phenyl, für unsubstituiertes oder durch Methoxy monosubstituiertes Biphenylyl oder für durch Trifluormethyl monosubstituiertes Phenoxyphenyl steht.

6. Verbindungen nach Anspruch 1 der Formeln I und Ia, worin einer der Substituenten $R_1$ und $R_2$ für Methyl und der andere für Methyl, Ethyl oder Allyl steht und $R_3$ für unsubstituiertes oder durch Fluor oder Chlor mono-oder disubstituiertes oder durch Methyl oder Methoxy monosubstituiertes Phenyl, für unsubstituiertes oder durch Methoxy monosubstituiertes 3-oder 4-Biphenylyl oder für (Trifluormethylphenoxy)-phenyl steht.

7. Verbindungen nach Anspruch 1 der Formeln I und Ia, worin $R_1$ und $R_2$ jeweils für Methyl stehen und $R_3$ für unsubstituiertes oder durch Fluor oder Chlor mono- oder disubstituiertes Phenyl oder für (Trifluormethylphenoxy)-phenyl steht.

8. Verbindungen der Formel I nach Anspruch 1, worin $R_1$ und $R_2$ jeweils für Methyl stehen und $R_3$ für unsubstituiertes oder durch Fluor oder Chlor mono- oder disubstituiertes Phenyl steht.

9. Eine Verbindung der Formel I nach Anspruch 1, ausgewählt aus der Reihe bestehend aus:
1,3-Dimethyl-5-[3,4-dichlorphenylcarbamoyl]-4(6)-oxo-6(4)-oxido-(1H,5H)-pyrimidiniumbetain,
1,3-Dimethyl-5-[2,4-difluorphenylcarbamoyl]-4(6)-oxo-6(4)-oxido-(1H,5H)-pyrimidiniumbetain,
1,3-Dimethyl-5-[4-biphenylylcarbamoy]-4(6)-oxo-6(4)-oxido-(1H, 5H)-pyrimidiniumbetain und
1,3-Dimethyl-5-[4-(4-trifluormethylphenoxy)phenylcarbamoyl]-4(6)-oxo-6(4) oxido-(1H,5H)-pyrimidiniumbetain.

10. Eine Verbindung der Formel I nach Anspruch 1 ausgewählt aus
1,3-Dimethyl-5-[phenylcarbamoyl]-4(6)-oxo-6(4)-oxido-(1H,5H)-pyrimidiniumbetain und
1,3-Dimethyl-5-[2-fluorphenylcarbamoyl]-4(6)-oxo-6(4)-oxido-(1H,5H)-pyrimidiniumbetain.

11. Verbindungen der Formel Ia nach Anspruch 1, worin $R_1$ und $R_2$ jeweils für Methyl stehen und $R_3$ für unsubstituiertes oder durch Fluor oder Chlor mono- oder disubstituiertes Phenyl oder für (Trifluormethylphenoxy)phenyl steht.

12. Eine Verbindung der Formel Ia nach Anspruch 1, ausgewählt aus der Reihe bestehend aus:
1,3-Dimethyl-5-[3,4-dichlorphenylcarbamoyl]-4,6-(1H,3H,5H)-pyrimidindion,
1,3-Dimethyl-5-[2,4-difluorphenylcarbamoyl]-4,6-(1H,3H,5H)-pyrimidindion,
1,3-Dimethyl-5-[2-fluorphenylcarbamoyl]-4,6-(1H,3H,5H)-pyrimidindion,
1,3-Dimethyl-5-[3,4-dichlorphenylcarbamoyl]-4,6-(1H,3H,5H)-pyrimidindion,
1,3-Dimethyl-5-[4-biphenylylcarbamoyl]-4,6-(1H,3H,5H)-pyrimidindion und
1,3-Dimethyl-5-[4-(4-trifluormethylphenoxy)phenylcarbamoyl]-4,6-(1H,3H,5H)-pyrimidindion.

13. Die Verbindung der Formel Ia nach Anspruch 1:
1,3-Dimethyl-5-[phenylcarbamoyl]-4,6-(1H,3H,5H)-pyrimidindion.

14. Verfahren zur Herstellung einer Verbindung der Formel I

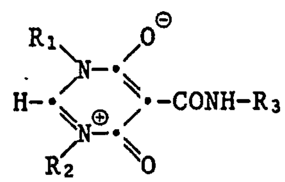

(I)

oder in ihrer hydrierten Form der Formel Ia

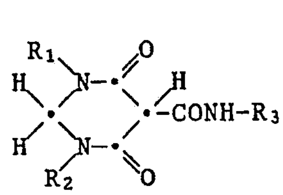

(Ia)

worin
$R_1$ und $R_2$ unabhängig voneinander für $C_1$-$C_6$-Alkyl, Allyl, $C_3$-$C_6$-Cycloalkyl oder Phenyl stehen; und $R_3$ für unsubstituiertes oder ein- oder mehrfach substituiertes Phenyl, Biphenylyl oder Phenoxyphenyl steht; wobei deren Substituenten ausgewählt sind aus der Gruppe Halogen, Cyano, $C_1$-$C_3$-Alkyl, $C_1$-$C_3$-Haloalkyl, $C_1$-$C_3$-Alkoxy, $C_1$-$C_3$-Haloalkoxy, $C_1$-$C_3$-Cyanoalkyl, Nitro, Amino oder durch $C_1$-$C_3$-Alkoxycarbonyl substituiertes $C_1$-$C_3$-Alkyl, unter Einschluss ihrer tautomeren Formen und physiologisch verträglichen Salze, dadurch

gekennzeichnet, dass man eine Verbindung der Formel II

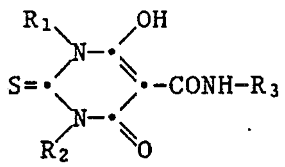

(II),

worin $R_1$, $R_2$ und $R_3$ die unter Formel I und Ia angegebenen Bedeutungen haben, entschwefelt.

15. Verfahren nach Anspruch 14, dadurch gekennzeichnet, dass man die Entschwefelung durch katalytische Hydrierung in einem reaktionsinerten Lösungsmittel oder Lösungsmittelgemisch bei Temperaturen in einem Bereich von 20 bis 180 °C vornimmt.

16. Verfahren nach Anspruch 15, dadurch gekennzeichnet, dass man die Hydrierung mit einem Trialkylzinnhydrid, Trialkylzinnhalogenid, Trialkylgermaniumhydrid, Trialkylgermaniumhalogenid, Alkylquecksilberhydrid oder Alkylquecksilberhalogenid durchführt und in den Fällen in denen man ein Halogenid einsetzt, zusätzlich in Gegenwart von $NaBH_4$ arbeitet.

17. Verfahren nach Anspruch 16, dadurch gekennzeichnet, dass man in Gegenwart eines Radikalinitiators arbeitet.

18. Verfahren nach Anspruch 15, dadurch gekennzeichnet, dass man die Hydrierung mit Tris(trimethylsilyl)-silan durchführt.

19. Verfahren nach Anspruch 18, dadurch gekennzeichnet, dass man in Gegenwart eines Radikalinitiators arbeitet.

20. Anthelmintisches Mittel, dadurch gekennzeichnet, dass es als aktive Komponente mindestens eine Verbindung der Formel I oder Ia, ein Tautomeres oder ein Salz davon gemäss Anspruch 1 neben Träger- und weiteren Hilfsstoffen enthält.

21. Verbindung der Formel I oder Ia nach Anspruch 1 zur Verwendung in einem Verfahren zur Bekämpfung von Wurmerkrankungen bei Warmblütern.

22. Verwendung einer Verbindung der Formel I oder Ia zur Herstellung eines Mittels zur Bekämpfung von Helminthen.

Patentansprüche für folgende Vertragsstaaten: ES, GR

1. Verfahren zur Herstellung von Verbindungen der Formel I

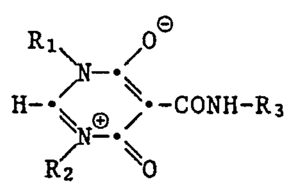

(I)

oder in ihrer hydrierten Form der Formel Ia

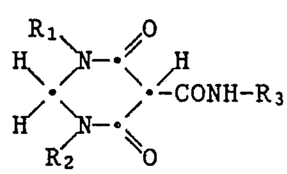

(Ia)

worin

$R_1$ und $R_2$ unabhängig voneinander für $C_1$-$C_6$-Alkyl, Allyl, $C_3$-$C_6$-Cycloalkyl oder Phenyl stehen; und $R_3$ für unsubstituiertes oder ein- oder mehrfach substituiertes Phenyl, Biphenylyl oder Phenoxyphenyl steht; wobei deren Substituenten ausgewählt sind aus der Gruppe Halogen, Cyano, $C_1$-$C_3$-Alkyl, $C_1$-$C_3$-Haloalkyl, $C_1$-$C_3$-Alkoxy, $C_1$-$C_3$-Haloalkoxy, $C_1$-$C_3$-Cyanoalkyl, Nitro, Amino oder durch $C_1$-$C_3$-Alkoxycarbonyl substituiertes $C_1$-$C_3$-Alkyl, unter Einschluss ihrer tautomeren Formen und physiologisch verträglichen Salze, dadurch gekennzeichnet, dass man eine Verbindung der Formel II

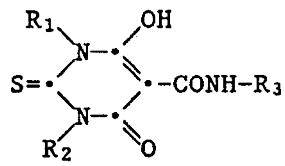

(II),

worin $R_1$, $R_2$ und $R_3$ die unter Formel I und Ia angegebenen Bedeutungen haben, entschwefelt.

2. Verfahren nach Anspruch 1 zur Herstellung von Verbindungen der Formeln I und Ia worin $R_1$ und $R_2$ unabhängig voneinander für Methyl, Ethyl, Isopropyl, Allyl oder Cyclopropyl stehen; und $R_3$ für ein- bis dreifach substituiertes Phenyl steht, wobei die Substituenten am Phenyl ausgewählt sind aus der Gruppe Fluor, Chlor, Brom, Methyl, Ethyl, Nitro, Amino, Trifluormethyl, Methoxy, Ethoxy, $CH_2CN$ oder $-CH(CH_3)-COOCH_3$.

3. Verfahren nach Anspruch 1 zur Herstellung von Verbindungen der Formeln I und Ia, worin $R_1$ und $R_2$ unabhängig voneinander für Methyl, Ethyl, Isopropyl, Allyl oder Cyclopropyl stehen; und $R_3$ für ein- bis dreifach substituiertes Phenoxyphenyl steht, wobei die Substituenten am Phenyl ausgewählt sind aus der Gruppe Fluor, Chlor, Brom, Methyl, Ethyl, Nitro, Amino, Trifluormethyl, Methoxy, Ethoxy, $CH_2CN$ oder $-CH(CH_3)COOCH_3$.

4. Verfahren nach Anspruch 1 zur Herstellung von Verbindungen der Formeln I und Ia, worin $R_1$ und $R_2$ unabhängig voneinander für Methyl, Ethyl, Isopropyl, Allyl oder Cyclopropyl stehen; und $R_3$ für ein- bis dreifach substituiertes Biphenylyl steht, wobei die Substituenten am Phenyl ausgewählt sind aus der Gruppe Fluor, Chlor, Brom, Methyl, Ethyl, Nitro, Amino, Trifluormethyl, Methoxy, Ethoxy, $CH_2CN$ oder $-CH(CH_3)COOCH_3$.

5. Verfahren nach Anspruch 1 zur Herstellung von Verbindungen der Formeln I und Ia, worin $R_1$ und $R_2$ unabhängig voneinander für Methyl, Ethyl oder Allyl stehen und $R_3$ für unsubstituiertes oder durch einen oder zwei Substituenten, ausgewählt aus der Gruppe Fluor, Chlor, Methyl und Methoxy, substituiertes Phenyl, für unsubstituiertes oder durch Methoxy monosubstituiertes Biphenylyl oder für durch Trifluormethyl monosubstituiertes Phenoxyphenyl steht.

6. Verfahren nach Anspruch 1 zur Herstellung von Verbindungen der Formeln I und Ia, worin einer der Substituenten $R_1$ und $R_2$ für Methyl und der andere für Methyl, Ethyl oder Allyl steht und $R_3$ für unsubstituiertes oder durch Fluor oder Chlor mono- oder disubstituiertes oder durch Methyl oder Methoxy monosubstituiertes Phenyl, für unsubstituiertes oder durch Methoxy monosubstituiertes 3- oder 4-Biphenylyl oder für (Trifluormethylphenoxy)-phenyl steht.

7. Verfahren nach Anspruch 1 zur Herstellung von Verbindungen der Formeln I und Ia, worin $R_1$ und $R_2$ jeweils für Methyl stehen und $R_3$ für unsubstituiertes oder durch Fluor oder Chlor mono- oder disubstituiertes Phenyl oder für (Trifluormethylphenoxy)-phenyl steht.

8. Verfahren nach Anspruch 1 zur Herstellung von Verbindungen der Formel I, worin $R_1$ und $R_2$ jeweils für Methyl stehen und $R_3$ für unsubstituiertes oder durch Fluor oder Chlor mono- oder disubstituiertes Phenyl steht.

9. Verfahren nach Anspruch 1 zur Herstellung einer Verbindung der Formel I, ausgewählt aus der Reihe bestehend aus:

1,3-Dimethyl-5-[3,4-dichlorphenylcarbamoyl]-4(6)-oxo-6(4)-oxido-(1H,5H)-pyrimidiniumbetain,
1,3-Dimethyl-5-[2,4-difluorphenylcarbamoyl]-4(6)-oxo-6(4)-oxido-(1H,5H)-pyrimidiniumbetain,
1,3-Dimethyl-5-[4-biphenylylcarbamoyl]-4(6)-oxo-6(4)-oxido-(1H,5H)-pyrimidiniumbetain und
1,3-Dimethyl-5-[4-(4-trifluormethylphenoxy)phenylcarbamoyl]-4(6)-oxo-6(4)-oxido-(1H,5H)-pyrimidiniumbetain.

10. Verfahren nach Anspruch 1 zur Herstellung einer Verbindung der Formel I, ausgewählt aus
1,3-Dimethyl-5-[phenylcarbamoyl]-4(6)-oxo-6(4)-oxido-(1H,5H)-pyrimidiniumbetain und
1,3-Dimethyl-5-[2-fluorphenylcarbamoyl]-4(6)-oxo-6(4)-oxido-(1H,5H)-pyrimidiniumbetain.

11. Verfahren nach Anspruch 1 zur Herstellung von Verbindungen der Formel Ia, worin $R_1$ und $R_2$ jeweils für Methyl stehen und $R_3$ für unsubstituiertes oder durch Fluor oder Chlor mono- oder disubstituiertes Phenyl oder für (Trifluormethylphenoxy)-phenyl steht.

12. Verfahren nach Anspruch 1 zur Herstellung einer Verbindung der Formel Ia, ausgewählt aus der Reihe bestehend aus:

1,3-Dimethyl-5-[3,4-dichlorphenylcarbamoyl]-4,6-(1H,3H,5H)-pyrimidindion,
1,3-Dimethyl-5-[2,4-difluorphenylcarbamoyl]-4,6-(1H,3H,5H)-pyrimidindion,
1,3-Dimethyl-5-[2-fluorphenylcarbamoyl]-4,6-(1H,3H,5H)-pyrimidindion,
1,3-Dimethyl-5-[3,4-dichlorphenylcarbamoyl]-4,6-(1H,3H,5H)-pyrimidindion,

1,3-Dimethyl-5-[4-biphenylylcarbamoyl]-4,6-(1H,3H,5H)-pyrimidindion und
1,3-Dimethyl-5-[4-(4-trifluormethylphenoxy)phenylcarbamoyl]-4,6-(1H,3H,5H)-pyrimidindion.

13. Verfahren nach Anspruch 1 zur Herstellung der Verbindung der Formel Ia:
1,3-Dimethyl-5-[phenylcarbamoyl]-4,6-(1H,3H,5H)-pyrimidindion.

14. Verfahren nach Anspruch 1, dadurch gekennzeichnet, dass man die Entschwefelung durch katalytische Hydrierung in einem reaktionsinerten Lösungsmittel oder Lösungsmittelgemisch bei Temperaturen in einem Bereich von 20 bis 180° C vornimmt.

15. Verfahren nach Anspruch 14, dadurch gekennzeichnet, dass man die Hydrierung mit einem Trialkylzinnhydrid, Trialkylzinnhalogenid, Trialkylgermaniumhydrid, Trialkylgermaniumhalogenid, Alkylquecksilberhydrid oder Alkylquecksilberhalogenid durchführt und in den Fällen in denen man ein Halogenid einsetzt, zusätzlich in Gegenwart von $NaBH_4$ arbeitet.

16. Verfahren nach Anspruch 15, dadurch gekennzeichnet, dass man in Gegenwart eines Radikalinitiators arbeitet.

17. Verfahren nach Anspruch 14, dadurch gekennzeichnet, dass man die Hydrierung mit Tris(trimethylsilyl)-silan durchführt.

18. Verfahren nach Anspruch 17, dadurch gekennzeichnet, dass man in Gegenwart eines Radikalinitiators arbeitet.

19. Verfahren zur Herstellung eines anthelmintischen Mittels, dadurch gekennzeichnet, dass man als aktive Komponente mindestens eine Verbindung der Formel I oder Ia, ein Tautomeres oder ein Salz davon gemäss Anspruch 1 mit Träger- und weiteren Hilfsstoffen vermischt.

20. Verwendung einer Verbindung der Formel I oder Ia zur Herstellung eines Mittels zur Bekämpfung von Helminthen.